# EUROPEAN PATENT SPECIFICATION

(11) **EP 4 252 663 B1**
(45) Date of publication and mention of the grant of the patent: **01.01.2025**
(21) Application number: 21897565.4
(22) Date of filing: 21.10.2021
(51) Int. Cl.: A61B 6/03, A61B 5/055, A61B 6/46, G06F 3/0481, G06T 19/00, G06T 7/00

(54) **IMAGE DISPLAY DEVICE, METHOD, AND PROGRAM**
BILDANZEIGEVORRICHTUNG, -VERFAHREN UND -PROGRAMM
DISPOSITIF, PROCÉDÉ ET PROGRAMME D'AFFICHAGE D'IMAGE

(30) Priority: 27.11.2020 JP 2020197514
(43) Date of publication of application: 04.10.2023
(73) Proprietor: FUJIFILM Corporation, Tokyo 106-8620 (JP)
(72) Inventor: ICHINOSE, Akimichi, Tokyo 106-8620 (JP); NAKAMURA, Keigo, Tokyo 106-8620 (JP)
(74) Representative: Klunker IP Patentanwälte PartG mbB
(86) International application number: PCT/JP2021/039013
(87) International publication number: WO 2022/113587

(56) References cited:
- JP-A- 2004 213 643
- JP-A- 2004 275 601
- JP-A- 2004 275 601
- US-A1- 2004 120 558
- US-A1- 2007 057 962
- US-A1- 2018 177 446
- US-A1- 2018 341 747
- US-A1- 2018 341 753

## Description

### BACKGROUND OF THE INVENTION

### 1. Field of the Invention

The present disclosure relates to an image display apparatus, method, and program.

### 2. Description of the Related Art

In recent years, advances in medical devices, such as computed tomography (CT) apparatuses and magnetic resonance imaging (MRI) apparatuses, have enabled image diagnosis using high-resolution medical images with higher quality. In particular, since a region of a lesion can be accurately specified by image diagnosis using CT images, MRI images, and the like, appropriate treatment is being performed based on the specified result.

In addition, image diagnosis is made by analyzing a medical image via computer-aided diagnosis (CAD) using a learning model in which machine learning is performed by deep learning or the like, and detecting diseased regions such as a lesion included in the medical image as regions of interest from the medical image. In this way, the analysis result generated by the analysis process via CAD is saved in a database in association with examination information, such as a patient name, gender, age, and a modality which has acquired a medical image, and provided for diagnosis. A doctor interprets a medical image by referring to a distributed medical image and analysis result in his or her own interpretation terminal. At this time, in the interpretation terminal, an annotation is superimposed and displayed on the region of interest including the disease included in the medical image based on the analysis result. An annotation is a general term for figures, characters, and symbols attached to a medical image. For example, figures such as a rectangle surrounding the region of interest and an arrow indicating the region of interest, and text such as the type and size of the disease are superimposed and displayed as annotations. A radiologist refers to the annotations added to the region of interest to create an interpretation report.

Incidentally, in a case where a plurality of lesions are included in the medical image, an annotation is added to each of the lesions. Therefore, in a case where the medical image to which the annotation is added is displayed, a plurality of annotations are superimposed and displayed on the medical image. In such a case where a plurality of annotations are superimposed and displayed, it is difficult to clearly grasp the position of the lesion indicated by the displayed annotations. Therefore, a method has been proposed in which a plurality of annotations are grouped and associated with a medical image in a unit of a group to clarify the association between the plurality of annotations and the medical image (see, for example, JP2013-132514A). Further, in the method described in JP2013-132514A, there has been also proposed a method of further associating a character string of a finding included in an interpretation report with a medical image and an annotation.

US2018/177446A1 discloses an image interpretation support apparatus and method. The apparatus includes a line-of-sight detecting unit that detects a line of sight of a person who reads the image and a gaze determining unit that determines, in accordance with a detection result obtained by the line-of-sight detecting unit, whether a candidate lesion portion in an image to be interpreted that is displayed on a display unit has been gazed at by the person who reads the image. The apparatus further includes a completion detecting unit that detects completion of image interpretation, by the person who reads the image, of the image to be interpreted that is displayed on the display unit, and a display control unit that, in a case where the completion detecting unit detects the completion of the image interpretation and the gaze determining unit determines that the candidate lesion portion has not been gazed at, switches from a first display state to a second display state.

US2004120558A1 discloses a computer assisted data reconciliation method and apparatus. JP2004275601A discloses an image management device and image display device. US2018341753A1 discloses a medical scan interface feature evaluating system. US2007057962A1 discloses an image processing method and computer readable medium for image processing.

### SUMMARY OF THE INVENTION

On the other hand, in a case of interpreting a medical image to which a plurality of annotations are added, annotations other than the annotations added to a region of attention in the medical image interfere with the interpretation for a radiologist. In a case where the method described in JP2013-132514A is used, it is possible to display only annotations included in a specific group, but some of them may include annotations that are not necessary for interpretation. In such a case, unnecessary annotations can be hidden and only necessary annotations can be displayed by performing an operation of clicking unnecessary annotations or clicking a region to which unnecessary annotations are added. However, since the radiologist interprets a large number of medical images every day, such an operation is very troublesome for the radiologist.

The present disclosure has been made in view of the above circumstances, and an object of the present disclosure is to enable a radiologist to refer only to an annotation about a region of attention without performing a troublesome operation.

According to an aspect of the present disclosure, there is provided an image display apparatus according to claim 1 of the appended claims.

In addition, in the image display apparatus according to the aspect of the present disclosure, the processor may be configured to switch to hide or weakly display the relevant information related to the regions of interest other than the region of attention.

In addition, in the image display apparatus according to the aspect of the present disclosure, the other information may be an observation condition of the medical image.

In addition, in the image display apparatus according to the aspect of the present disclosure, the processor may be configured to: display the medical image in which all relevant information is hidden; and display the relevant information related to the region of attention in a case where the region of attention is specified based on the detection result of the other operation.

In addition, in the image display apparatus according to the aspect of the present disclosure, the other operation may be an operation of inputting a comment on findings related to the medical image, and the processor may be configured to specify a region of interest related to a term included in the comment on findings as the region of attention.

In addition, in the image display apparatus according to the aspect of the present disclosure, the other operation may be an operation of further adding the relevant information to the region of interest included in the medical image, and the processor may be configured to specify the region of interest to which the relevant information is added as the region of attention.

In addition, in the image display apparatus according to the aspect of the present disclosure, the other operation may be an operation of changing a size of the medical image with reference to a designated region of interest, and the processor may be configured to specify the designated region of interest as the region of attention.

In addition, in the image display apparatus according to the aspect of the present disclosure, the processor may be configured to switch to hide the relevant information superimposed on the region of attention by the other operation.

In addition, in the image display apparatus according to the aspect of the present disclosure, the specification information may include only the other information.

In addition, in the image display apparatus according to the aspect of the present disclosure, the relevant information may include an annotation related to the region of interest.

In addition, in the image display apparatus according to the aspect of the present disclosure, the annotation may include at least one of a recognition result of the region of interest or character information related to the region of interest.

In addition, in the image display apparatus according to the aspect of the present disclosure, the region of interest may be a lesion or an organ included in the medical image.

According to another aspect, there is provided an image display method according to claim 13 of the appended claims.

According to another aspect, there is provided a computer-readable storage medium according to claim 14 of the appended claims.

According to the aspects of the present disclosure, the radiologist can refer only to the annotation about the region of attention without performing a troublesome operation.

### BRIEF DESCRIPTION OF THE DRAWINGS

Fig. 1 is a diagram showing a schematic configuration of a medical information system to which an image display apparatus according to an embodiment of the present disclosure is applied.
Fig. 2 is a diagram showing a schematic configuration of the image display apparatus according to the present embodiment.
Fig. 3 is a functional configuration diagram of the image display apparatus according to the present embodiment.
aFig. 4 is a diagram schematically showing a relationship between a target image and an annotation.
Fig. 5 is a diagram showing an annotation added to tomographic image groups shown in Fig. 4.
Fig. 6 is a diagram showing a display screen.
Fig. 7 is a diagram showing a property input window.
Fig. 8 is a diagram showing a comment-on-findings input window.
Fig. 9 is a diagram showing a display screen on which an annotation is displayed.
Fig. 10 is a diagram showing a display screen on which annotations are displayed.
Fig. 11 is a diagram showing a display screen on which an annotation is displayed.
Fig. 12 is a diagram for describing specification of a region of attention.
Fig. 13 is a diagram showing an enlarged target image.
Fig. 14 is a diagram showing a tomographic image on which the annotation is displayed.
Fig. 15 is a diagram showing a tomographic image on which the annotations are displayed.
Fig. 16 is a diagram showing a tomographic image on which the annotation is displayed.
Fig. 17 is a diagram showing a tomographic image on which the annotation is displayed.
Fig. 18 is a flowchart showing a process performed in the present embodiment.
Fig. 19 is a diagram showing a tomographic image in which a part of the annotations is weakly displayed.
Fig. 20 is a diagram showing a tomographic image in which a part of the annotations is hidden.
Fig. 21 is a diagram showing a tomographic image in which a part of the annotation is hidden.
Fig. 22 is a diagram showing a tomographic image in which a part of the annotation is hidden.
Fig. 23 is a diagram showing a state in which a mask representing a region extraction result as relevant information is superimposed and displayed on an image.
Fig. 24 is a diagram showing adjacent regions of interest.
Fig. 25 is a diagram showing a tomographic image in which a part of the annotation is hidden.

### DESCRIPTION OF THE PREFERRED EMBODIMENTS

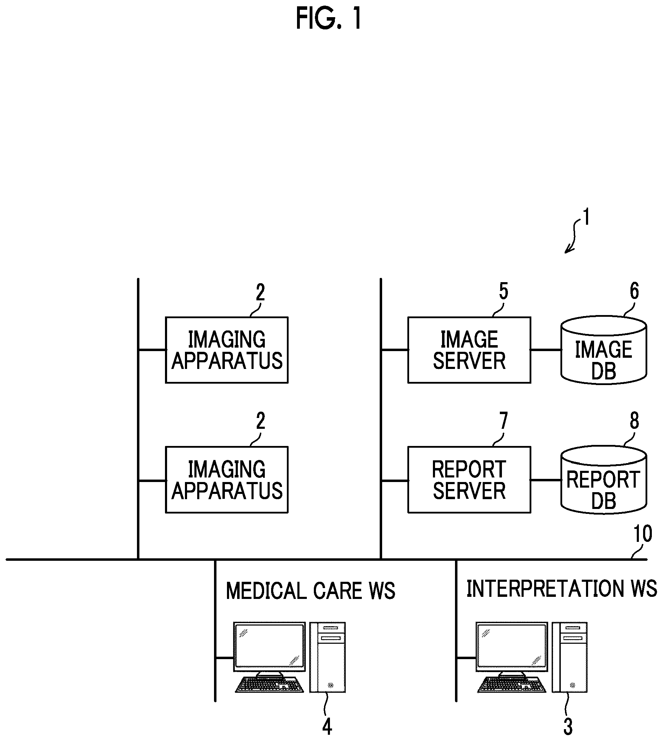

Hereinafter, embodiments of the present disclosure will be described with reference to the drawings. First, a configuration of a medical information system 1 to which an image display apparatus according to the present embodiment is applied will be described. Fig. 1 is a diagram showing a schematic configuration of the medical information system 1. The medical information system 1 shown in Fig. 1 is, based on an examination order from a doctor in a medical department using a known ordering system, a system for imaging an examination target part of a subject, storing a medical image acquired by the imaging, interpreting the medical image by a radiologist and creating an interpretation report, and viewing the interpretation report and observing the medical image to be interpreted in detail by the doctor in the medical department that is a request source.

As shown in Fig. 1, in the medical information system 1, a plurality of imaging apparatuses 2, a plurality of interpretation workstations (WSs) 3 that are interpretation terminals, a medical care WS 4, an image server 5, an image database (hereinafter referred to as an image DB) 6, a report server 7, and a report database (hereinafter referred to as a report DB) 8 are communicably connected to each other through a wired or wireless network 10.

Each apparatus is a computer on which an application program for causing each apparatus to function as a component of the medical information system 1 is installed. The application program is stored in a storage apparatus of a server computer connected to the network 10 or in a network storage in a state in which it can be accessed from the outside, and is downloaded to and installed on the computer in response to a request. Alternatively, the application program is recorded on a recording medium, such as a digital versatile disc (DVD) and a compact disc read only memory (CD-ROM), and distributed, and is installed on the computer from the recording medium.

The imaging apparatus 2 is an apparatus (modality) that generates a medical image showing a diagnosis target part of the subj ect by imaging the diagnosis target part. Specifically, examples of the modality include a simple X-ray imaging apparatus, a CT apparatus, an MRI apparatus, a positron emission tomography (PET) apparatus, and the like. The medical image generated by the imaging apparatus 2 is transmitted to the image server 5 and is saved in the image DB 6.

The interpretation WS 3 is a computer used by, for example, a radiologist of a radiology department to interpret a medical image and to create an interpretation report, and encompasses an image display apparatus according to the present embodiment. In the interpretation WS 3, a viewing request for a medical image to the image server 5, various image processing for the medical image received from the image server 5, display of the medical image, input reception of comments on findings regarding the medical image, and the like are performed. In the interpretation WS 3, creation of an interpretation report, a registration request and a viewing request for the interpretation report to the report server 7, display of the interpretation report received from the report server 7, and the like are performed. The above processes are performed by the interpretation WS 3 executing software programs for respective processes.

The medical care WS 4 is a computer used by a doctor in a medical department to observe an image in detail, view an interpretation report, create an electronic medical record, and the like, and is configured to include a processing apparatus, a display apparatus such as a display, and an input apparatus such as a keyboard and a mouse. In the medical care WS 4, a viewing request for the image to the image server 5, display of the image received from the image server 5, a viewing request for the interpretation report to the report server 7, and display of the interpretation report received from the report server 7 are performed. The above processes are performed by the medical care WS 4 executing software programs for respective processes.

The image server 5 is a general-purpose computer on which a software program that provides a function of a database management system (DBMS) is installed. The image server 5 comprises a storage in which the image DB 6 is configured. This storage may be a hard disk apparatus connected to the image server 5 by a data bus, or may be a disk apparatus connected to a storage area network (SAN) or a network attached storage (NAS) connected to the network 10. In a case where the image server 5 receives a request to register a medical image from the imaging apparatus 2, the image server 5 prepares the medical image in a format for a database and registers the medical image in the image DB 6.

Image data of the medical image acquired by the imaging apparatus 2 and accessory information are registered in the image DB 6. The accessory information includes, for example, an image identification (ID) for identifying each medical image, a patient ID for identifying a subject, an examination ID for identifying an examination, a unique ID (unique identification (UID)) allocated for each medical image, examination date and examination time at which a medical image is generated, the type of imaging apparatus used in an examination for acquiring a medical image, patient information such as the name, age, and gender of a patient, an examination part (an imaging part), imaging information (an imaging protocol, an imaging sequence, an imaging method, imaging conditions, the use of a contrast medium, and the like), and information such as a series number or a collection number in a case where a plurality of medical images are acquired in one examination.

In addition, in a case where the viewing request from the interpretation WS 3 and the medical care WS 4 is received through the network 10, the image server 5 searches for a medical image registered in the image DB 6 and transmits the searched for medical image to the interpretation WS 3 and to the medical care WS 4 that are request sources.

The report server 7 incorporates a software program for providing a function of a database management system to a general-purpose computer. In a case where the report server 7 receives a request to register the interpretation report from the interpretation WS 3, the report server 7 prepares the interpretation report in a format for a database and registers the interpretation report in the report DB 8.

In the report DB 8, an interpretation report created by the radiologist using the interpretation WS 3 is registered. The interpretation report may include, for example, a medical image to be interpreted, an image ID for identifying the medical image, a radiologist ID for identifying the radiologist who performed the interpretation, a disease name, disease position information, information for accessing a medical image, and the like.

Further, in a case where the report server 7 receives the viewing request for the interpretation report from the interpretation WS 3 and the medical care WS 4 through the network 10, the report server 7 searches for the interpretation report registered in the report DB 8, and transmits the searched for interpretation report to the interpretation WS 3 and to the medical care WS 4 that are request sources.

In the present embodiment, the diagnosis target is the thoracoabdominal region of a human body, and the medical image is a three-dimensional CT image consisting of a plurality of tomographic images including the thoracoabdominal region. Then, in the interpretation WS 3, the radiologist interprets the CT image to create an interpretation report including comments on findings about diseases such as lungs and livers included in the thoracoabdominal region. The medical image is not limited to the CT image, and any medical image such as an MRI image and a simple X-ray image acquired by a simple X-ray imaging apparatus can be used.

In the present embodiment, in creating the interpretation report, a medical image is analyzed and a region of interest included in the medical image is detected. The radiologist interprets the image while referring to the detection result. The detection of the region of interest will be described later.

The network 10 is a wired or wireless local area network that connects various apparatuses in a hospital to each other. In a case where the interpretation WS 3 is installed in another hospital or clinic, the network 10 may be configured to connect local area networks of respective hospitals through the Internet or a dedicated line.

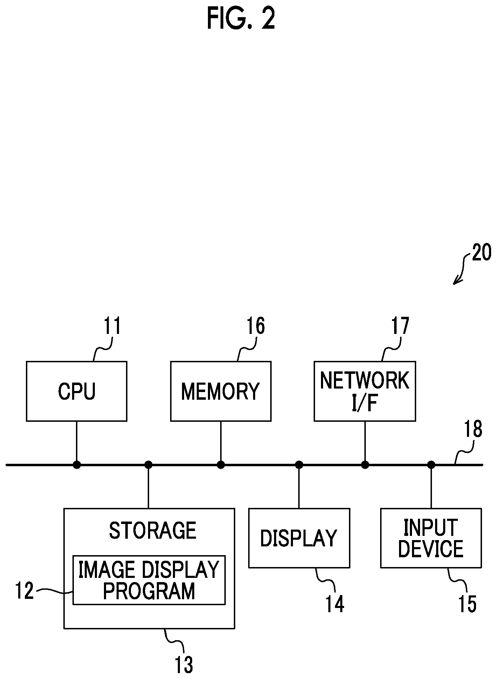

Next, the image display apparatus according to the present embodiment will be described. Fig. 2 illustrates a hardware configuration of the image display apparatus according to the present embodiment. As shown in Fig. 2, an image display apparatus 20 includes a central processing unit (CPU) 11, a non-volatile storage 13, and a memory 16 as a temporary storage area. Further, the image display apparatus 20 includes a display 14 such as a liquid crystal display, an input device 15 consisting of a pointing device such as a keyboard and a mouse, and a network interface (I/F) 17 connected to the network 10. The CPU 11, the storage 13, the display 14, the input device 15, the memory 16, and the network I/F 17 are connected to a bus 18. The CPU 11 is an example of a processor in the present disclosure.

The storage 13 is realized by a hard disk drive (HDD), a solid state drive (SSD), a flash memory, and the like. An image display program 12 is stored in the storage 13 as the storage medium. The CPU 11 reads out the image display program 12 from the storage 13, loads the read-out program into the memory 16, and executes the loaded image display program 12.

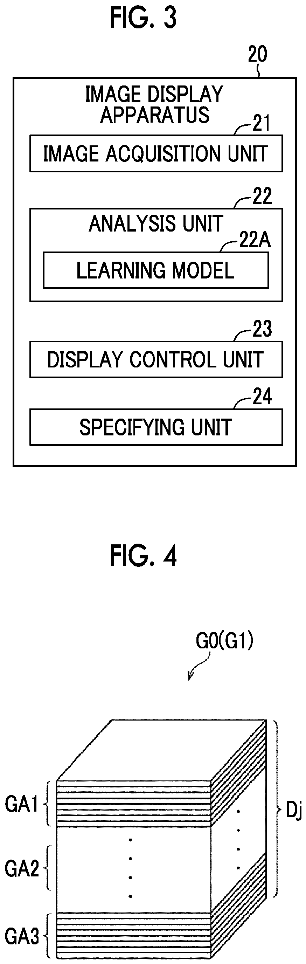

Next, a functional configuration of the image display apparatus according to the present embodiment will be described. Fig. 3 is a diagram showing a functional configuration of the image display apparatus according to the present embodiment. As shown in Fig. 3, the image display apparatus 20 comprises an image acquisition unit 21, an analysis unit 22, a display control unit 23, and a specifying unit 24. Then, the CPU 11 executes the image display program 12, so that the CPU 11 functions as the image acquisition unit 21, the analysis unit 22, the display control unit 23, and the specifying unit 24.

The image acquisition unit 21 acquires a medical image to be interpreted for creating an interpretation report from the image server 5 according to an instruction from the input device 15 by the radiologist who is an operator. A medical image to be interpreted is referred to as a target image G0 in the following description. In addition, a plurality of images may be acquired in one examination. Since all the images acquired in one examination are interpreted, there may be a plurality of target images G0. In addition, a plurality of images of a patient to be examined for interpreting the target image G0 may be acquired and saved in the image server 5 in the past. In the present embodiment, in a case of interpreting the target image G0 of the patient to be examined, all the images acquired by imaging the patient to be examined, including the target image G0, are acquired from the image server 5. In the following description, among all images of the patient acquired from the image server 5, images other than the target image G0 are acquired before the target image G0 in a timely manner, and thus are referred to as past images GP0.

In the present embodiment, the target image G0 is a three-dimensional CT image consisting of a plurality of tomographic images acquired by imaging the thoracoabdominal region of the patient to be examined. Further, in a case where a plurality of target images G0 are acquired in one examination, each target image G0 may include an X-ray image acquired by simple X-ray imaging in addition to the CT image. Moreover, the past image GP0 may also include an X-ray image acquired by simple X-ray imaging in addition to the CT image.

The analysis unit 22 detects a region of the abnormal shadow included in the target image G0 as a region of interest, and derives an annotation for the detected region of interest. The analysis unit 22 detects regions of shadows of a plurality of types of diseases as regions of interest from the target image G0 using a known computer-aided diagnosis (that is, CAD) algorithm, and derives annotations by deriving the properties of the regions of interest.

In order to detect the regions of interest and derive the annotations, the analysis unit 22 has a learning model 22Ain which machine learning is performed to detect abnormal shadows of a plurality of types of diseases as the regions of interest from the target image G0, and further derive properties of the abnormal shadows.

The learning model 22A consists of a convolutional neural network (CNN) in which deep learning has been performed using supervised training data to discriminate whether or not each pixel (voxel) in the target image G0 represents a shadow of various diseases or an abnormal shadow.

The learning model 22A is constructed by training CNN using, for example, supervised training data consisting of supervised training images that include abnormal shadows, a region of the abnormal shadows in the supervised training image, and correct answer data representing the properties of the abnormal shadows, and supervised training data consisting of supervised training images that do not include abnormal shadows. The learning model 22A derives the confidence degree (likelihood) indicating that each pixel in the medical image is an abnormal shadow, and detects a region consisting of pixels whose confidence degree is equal to or higher than a predetermined threshold value as a region of interest. Here, the confidence degree is a value of 0 or more and 1 or less. Further, the learning model 22A derives the properties of the detected region of interest. The properties include the position and the size of the region of interest, the type of disease, and the like.

The learning model 22A may detect a region of interest from a three-dimensional target image G0, or may detect a region of interest from each of a plurality of tomographic images constituting the target image G0. Further, as the learning model 22A, any learning model such as, for example, a support vector machine (SVM) can be used in addition to the convolutional neural network.

The analysis unit 22 derives, as annotations, a figure surrounding the region of interest detected by the learning model, text representing the property, a line connecting the figure and the text, and the like. The annotation is derived as an image of a channel different from that of the target image G0 and is added to the target image G0. Therefore, in a case where the image of the channel of the annotation is superimposed and displayed on the target image G0, the annotation is superimposed and displayed on the target image G0.

Fig. 4 is a diagram schematically showing a relationship between the target image G0 and the annotation. As shown in Fig. 4, the target image G0 includes a plurality of tomographic images Dj. Further, it is assumed that an abnormal shadow is detected as a region of interest in each of tomographic image groups GA1, GA2, and GA3 of the plurality of tomographic images Dj, and an annotation is added to the detected region of interest. In the present embodiment, the tomographic image Dj represents an axial cross section of a human body, but the present disclosure is not limited thereto.

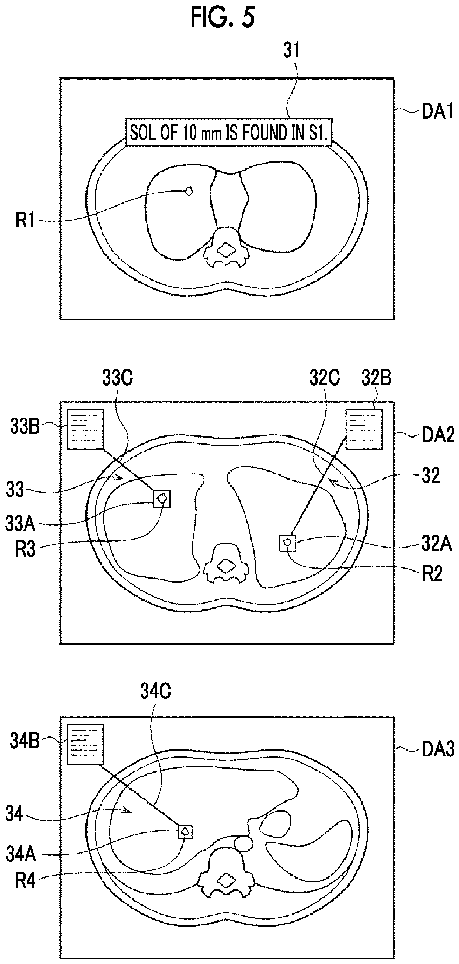

Fig. 5 is a diagram showing an annotation added to the tomographic image groups GA1 to GA3 shown in Fig. 4. As shown in Fig. 5, in a tomographic image DA1 included in the tomographic image group GA1, a region of interest R1 is detected, and an annotation 31 is added to the region of interest R1. The annotation 31 is character information related to the region of interest R1, and the content thereof is "SOL of 10 mm is found in S1". The SOL is an abbreviation for Space Occupying Lesion. The annotation 31 is added to all the tomographic images in which the region of interest R1 is detected, which are included in the tomographic image group GA1.

In addition, in a tomographic image DA2 included in the tomographic image group GA2, a region of interest R2 is detected in the left lung, and a region of interest R3 is detected in the right lung. In the tomographic image DA2, an annotation 32 and an annotation 33 are added to the region of interest R2 of the left lung and the region of interest R3 of the right lung, respectively. The annotation 32 consists of a figure 32A surrounding a region of interest of the left lung included in the tomographic image DA2, a text 32B that represents the properties of the region of interest surrounded by the figure 32A, and a leader line 32C connecting the figure 32A and the text 32B. The annotation 33 consists of a figure 33A surrounding a region of interest of the right lung included in the tomographic image DA2, a text 33B that represents the properties of the region of interest surrounded by the figure 33A, and a leader line 33C connecting the figure 33A and the text 33B. The texts 32B and 33B related to the region of interest are, for example, the position, the size, the property, or the like of the region of interest. The annotations 32 and 33 are added to all the tomographic images in which the regions of interest R2 and R3 are detected, respectively, which are included in the tomographic image group GA2.

Further, in a tomographic image DA3 included in the tomographic image group GA3, a region of interest R4 included in the liver is detected, and an annotation 34 is added to the region of interest R4 of the liver. The annotation 34 consists of a figure 34A surrounding a region of interest of the liver included in the tomographic image DA3, a text 34B that represents the properties of the region of interest surrounded by the figure 34A, and a leader line 34C connecting the figure 34A and the text 34B. The annotation 34 is added to all the tomographic images in which the region of interest R4 is detected, which are included in the tomographic image group GA3.

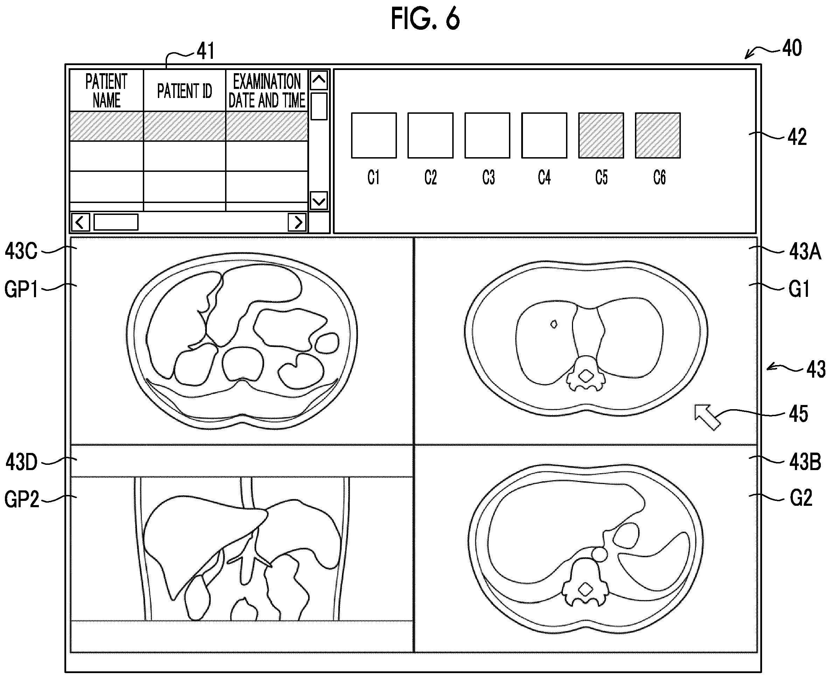

The display control unit 23 displays an image of the patient to be examined including the target image G0 and the past image GP0 on the display 14. Fig. 6 is a diagram showing a display screen of an image. As shown in Fig. 6, a display screen 40 includes an examination list area 41 for displaying an examination list, a thumbnail image area 42 for displaying thumbnail images of the target image G0 included in the selected examination and past images of the patient to be examined, and an image display area 43 for displaying an image.

In the examination list area 41, a patient name, a patient ID, and an examination date and time are displayed for each examination. The operator can select the patient to be examined by clicking on a desired examination list. It should be noted that, in Fig. 6, diagonal lines are added to the rows of the patient to be examined selected in the examination list area 41.

In the thumbnail image area 42, thumbnail images of the target images G0 of the selected patient to be examined and representative images of the past images GP0 are displayed. Here, assuming that two target images G1 and G2 and four past images GP1 to GP4 are acquired for the patient to be examined, as shown in Fig. 6, six thumbnail images C1 to C6 are displayed in the thumbnail image area 42. In a case where the image is a three-dimensional image, a minified image of a tomographic image of a predetermined tomographic plane included in the three-dimensional image can be used as a representative image. In the case of a simple X-ray image, the minified image can be used as a representative image. In addition, in the present embodiment, it is assumed that the thumbnail images C1 and C2 are the thumbnail images of the target images G1 and G2, and the thumbnail images C3 to C6 are the thumbnail images of the past images GP1 to GP4. Note that it is assumed that the target image G1 corresponds to the target image G0 shown in Fig. 4, abnormal shadows are detected as regions of interest in the tomographic image groups GA1, GA2, and GA3 of the plurality of tomographic images Dj included in the target image G1, and annotations are added to the detected regions of interest.

The image selected in the thumbnail image area 42 is displayed in the image display area 43. As shown in Fig. 6, the image display area 43 includes four display areas 43A to 43D. In Fig. 6, representative images of the four images selected from the six thumbnail images C1 to C6 are displayed in the display areas 43Ato 43D of the image display area 43, respectively. In addition, in the thumbnail image area 42, diagonal lines are added to the thumbnail images other than the images displayed in the image display area 43 in order to clearly indicate that the thumbnail images are not displayed in the image display area 43. In the present embodiment, since the thumbnail images C1 and C2 correspond to the target images G1 and G2, respectively, the target images G1 and G2 are displayed in the display areas 43A and 43B, respectively. It is also assumed that the past images GP1 and GP2 are displayed in the display areas 43C and 43D.

Although annotations are added to the target image G1 as shown in Fig. 4, the annotations are not superimposed and displayed on the representative image of the target image G1 displayed on the initial display screen 40 immediately after being displayed on the display 14.

In the present embodiment, the radiologist interprets the target image G1 displayed on the display screen 40 to confirm the region of interest detected by the analysis unit 22, or newly specify an abnormal shadow that the analysis unit 22 could not detect. At this time, the radiologist can perform a paging operation of switching the tomographic plane of the target image G1 displayed in the display area 43A by moving a mouse cursor 45 to the display area 43A and rotating the mouse wheel. Further, the radiologist can perform an operation of changing the size of the displayed target image G1 by performing a predetermined size change operation using the input device 15. Examples of the size change operation include an operation of moving the mouse cursor 45 to a reference position where the size change is desired in the target image G1, and rotating the mouse wheel while pressing the ctrl key. In this case, the size of the target image G1 being displayed is changed around the cursor position. A paging operation and a size change operation are examples of other operations related to the display of the medical image.

In addition, as necessary, the radiologist can perform an operation of adding tag information indicating the property of the newly found abnormal shadow to the target image, or an operation of inputting a comment on findings about the target image G1. The operation of adding the tag information and the operation of inputting the comment on findings are examples of other operations related to the interpretation of the medical image.

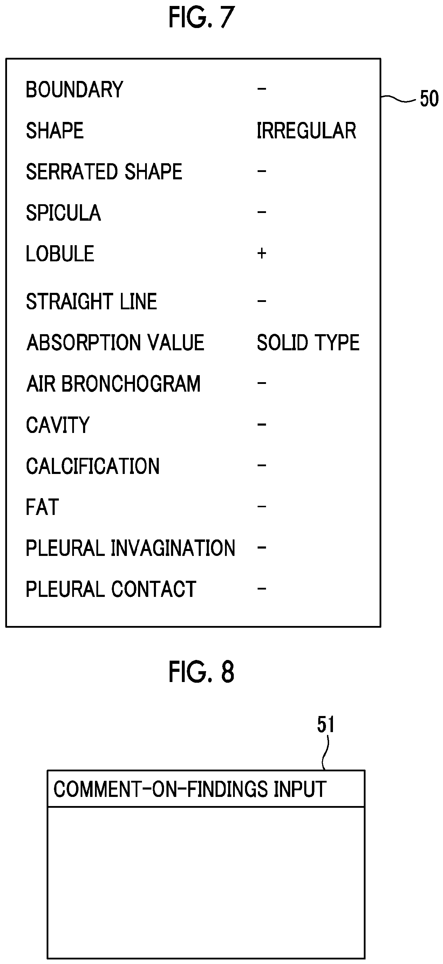

The operation of adding the tag information indicating the property to the target image is an operation of displaying a property input window 50 shown in Fig. 7 in a pop-up manner by, for example, specifying a newly found abnormal shadow with the mouse cursor 45 and right-clicking the specified abnormal shadow. In the property input window 50, for example, items of a boundary, a shape, a serrated shape, a spicula, a lobule, a straight line, an absorption value, an air bronchogram, a cavity, a calcification, a fat, a pleural invagination, and a pleural contact are shown as the properties of the abnormal shadow of the lung. Negative (-) or positive (+) can be selected for the items of the boundary, serrated shape, spicula, lobule, straight line, air bronchogram, cavity, calcification, fat, pleural invagination, and pleural contact. As for the shape item, irregular and well shape items can be selected. For the absorption value, a solid type or a frosted glass type can be selected. In addition, the operation of inputting the comment on findings is an operation of displaying a comment-on-findings input window 51 shown in Fig. 8 in a pop-up manner by right-clicking the mouse on the displayed target image.

The specifying unit 24 specifies a region of attention that the radiologist pays attention to among a plurality of regions of interest included in the target image. In the present embodiment, the specifying unit 24 specifies at least one region of attention based on specification information including other information other than information dedicated to specifying regions of attention. In the present embodiment, it is assumed that a detection result of another operation related to the display or interpretation of the medical image is used as other information. The specifying unit 24 specifies the region of attention based on specification information including detection results of other operations as other information. In addition, examples of the information dedicated to specifying the region of attention include a detection result of an operation of clicking the region of attention using a mouse.

For example, in the interpretation of the target image G1, in a case where the radiologist finds an abnormal shadow in a certain tomographic image, a paging operation for repeatedly interpreting the tomographic images before and after the tomographic image including the abnormal shadow is performed. The paging operation for repeatedly interpreting the images is an operation of repeatedly switching the tomographic plane back and forth in the body axis direction of the human body by rotating the mouse wheel back and forth. In a case where such a paging operation is detected and the tomographic image repeatedly displayed during the paging operation includes an annotation, the specifying unit 24 specifies a region of interest to which the annotation is added as the region of attention that the radiologist pays attention to.

For example, in a case where the paging operation is performed in the vicinity of the tomographic image group GA1 of the target image G1 shown in Fig. 4, the specifying unit 24 specifies the region of interest R1 included in the tomographic image DA1 shown in Fig. 5 as the region of attention. Further, in a case where the paging operation is performed in the vicinity of the tomographic image group GA2 in the target image G1 shown in Fig. 4, the specifying unit 24 specifies the region of interest R2 and the region of interest R3 included in the tomographic image DA2 shown in Fig. 5 as the regions of attention. Further, in a case where the paging operation is performed in the vicinity of the tomographic image group GA3 in the target image G1 shown in Fig. 4, the specifying unit 24 specifies the region of interest R4 included in the tomographic image DA3 shown in Fig. 5 as the region of attention.

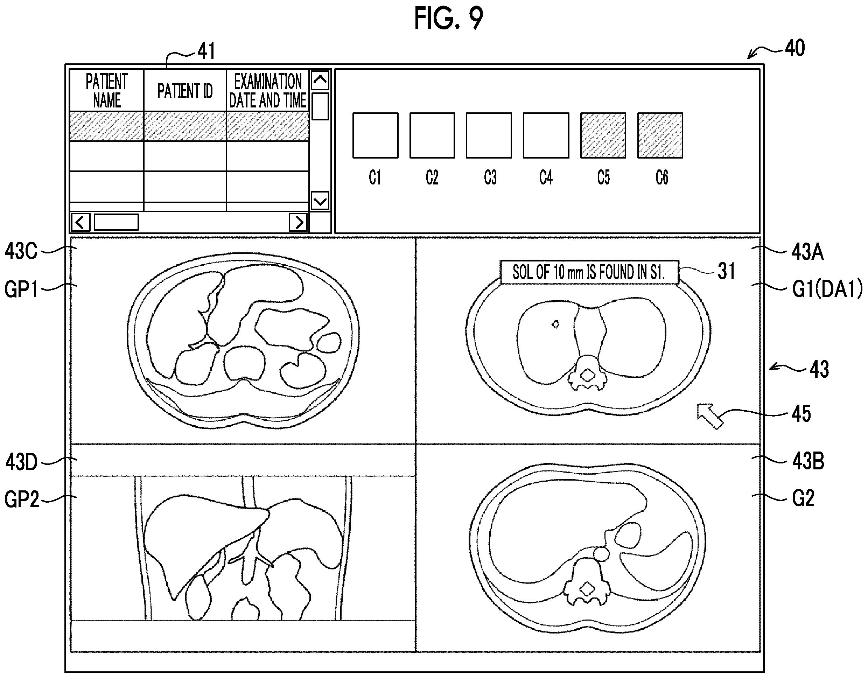

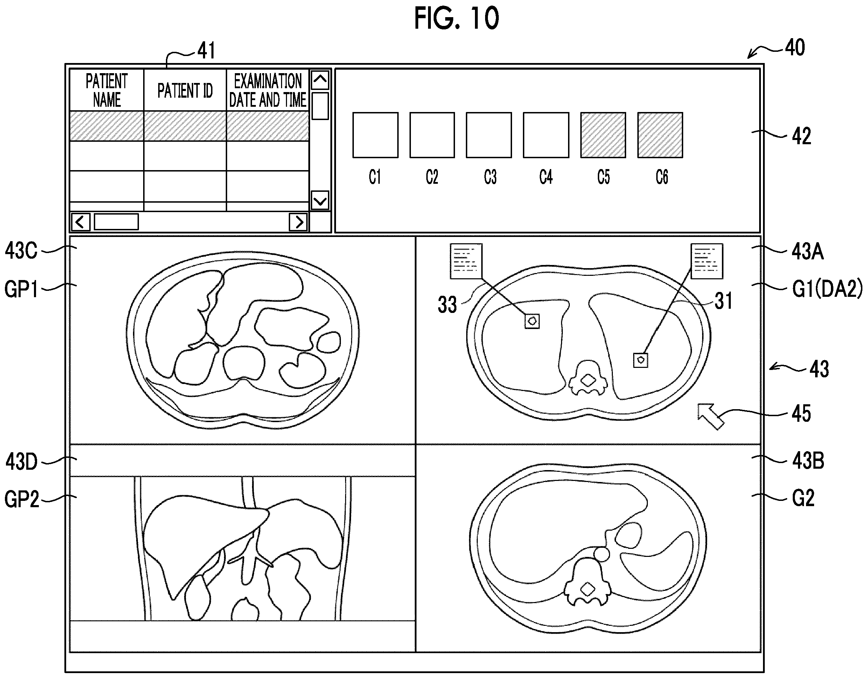

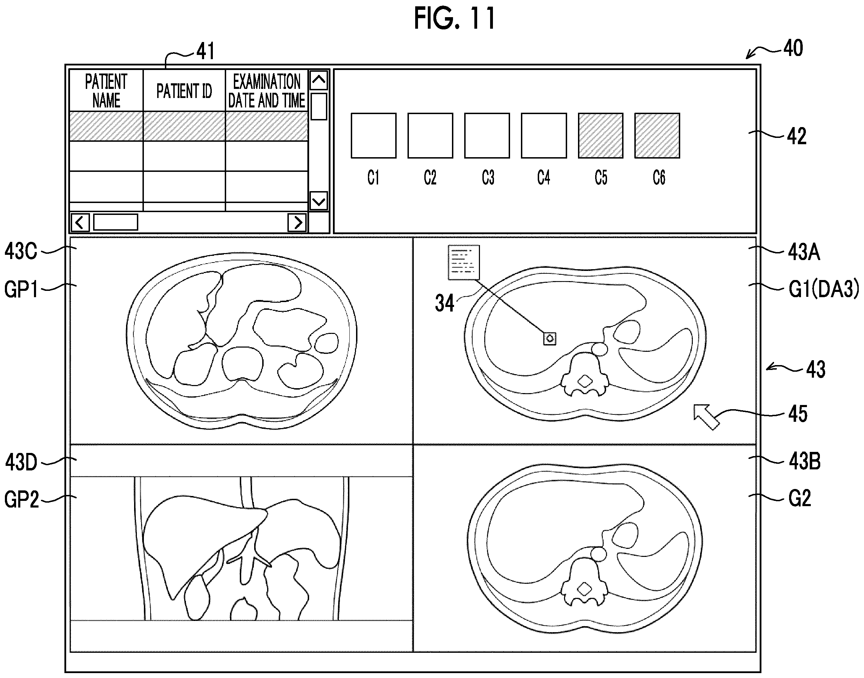

The display control unit 23 displays an annotation added to the region of attention specified by the specifying unit 24 on the display screen 40. That is, in a case where the paging operation is performed in the vicinity of the tomographic image group GA1 in the target image G1 displayed in the display area 43A, the annotation 31 is superimposed and displayed on the target image G1 (the tomographic image DA1) as shown in Fig. 9. In addition, in a case where the paging operation is performed in the vicinity of the tomographic image group GA2 in the target image G1, the annotations 32 and 33 are superimposed and displayed on the target image G1 (the tomographic image DA2) as shown in Fig. 10. In a case where the paging operation is performed in the vicinity of the tomographic image group GA3 in the target image G1, the annotation 34 is superimposed and displayed on the target image G1 (the tomographic image DA3) as shown in Fig. 11.

In addition, in a case where the paging operation is stopped, the display control unit 23 may continue to display the annotation or may hide the annotation. Alternatively, the annotation may be displayed for a predetermined time and then switched to be hidden.

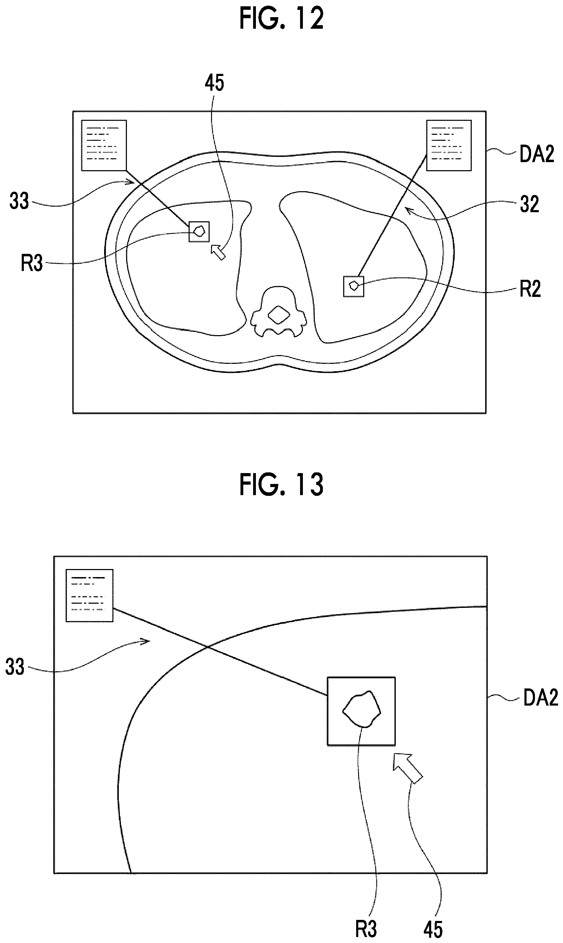

On the other hand, in a case where the radiologist finds the abnormal shadow in the target image G1, the radiologist may enlarge and display the abnormal shadow. In that case, the radiologist performs an operation of enlarging the displayed target image G1. For example, in the target image G1 (the tomographic image DA2) on which the annotations 32 and 33 are superimposed and displayed as shown in Fig. 10, in a case where the radiologist desires to enlarge the target image G1 with reference to the region of interest R3 to which the annotation 33 is added, as shown in Fig. 12, the radiologist moves the mouse cursor 45 to the region of interest R3 and rotates the mouse wheel while pressing a specific key such as the ctrl key. The specifying unit 24 specifies the region of interest R3 where the mouse cursor 45 is located as the region of attention based on the detection result of such an operation of changing the size.

The display control unit 23 displays an annotation added to the region of attention specified by the specifying unit 24 on the display screen 40. Here, as shown in Fig. 12, in a case where two regions of interest R2 and R3 are included in the tomographic image DA2 being displayed and the annotations 32 and 33 are superimposed and displayed on each of the regions of interest R2 and R3, it is assumed that the region of interest R3 is specified as the region of attention. In this case, the display control unit 23 displays only the annotation 33 for the region of interest R3 and hides the annotation 32 for the region of interest R2. Accordingly, as shown in Fig. 13, the target image G1 (the tomographic image DA2) enlarged centering on the region of interest R3 is displayed in the display area 43A, the annotation 32 is hidden, and only the annotation 33 added to the region of interest R3 is displayed.

In addition, in a case where the operation of changing the size is stopped, the display control unit 23 may continue to display the annotation or may hide the annotation. Alternatively, the annotation may be displayed for a predetermined time and then switched to be hidden.

In addition, in a case where the radiologist finds an abnormal shadow in the target image G1, the radiologist may display the comment-on-findings input window 51 shown in Fig. 8 in a pop-up manner and input a comment on findings about the abnormal shadow. The specifying unit 24 may specify a region of attention based on a detection result of an operation of inputting a comment on findings by the radiologist. Specifically, the specifying unit 24 may analyze the input comment on findings and specify a region of interest corresponding to the term included in the comment on findings as the region of attention. Here, as shown in Fig. 12, in a case where two regions of interest R2 and R3 are included in the tomographic image DA2 being displayed and the annotations 32 and 33 are superimposed and displayed on each of the regions of interest R2 and R3, it is assumed that the input comment on findings is "a nodule is seen in the left lung". The specifying unit 24 analyzes the comment on findings and specifies a part "left lung". The specifying unit 24 specifies the region of interest R2 of the left lung included in the tomographic image DA2 as the region of attention based on the specified term "left lung". Note that the specifying unit 24 may specify a term "nodule" included in the comment on findings, refer to the description of the texts 32B and 33B of the annotations 32 and 33 added to the regions of interest R2 and R3, and specify a region of interest to which an annotation including a description regarding a nodule is added as the region of attention.

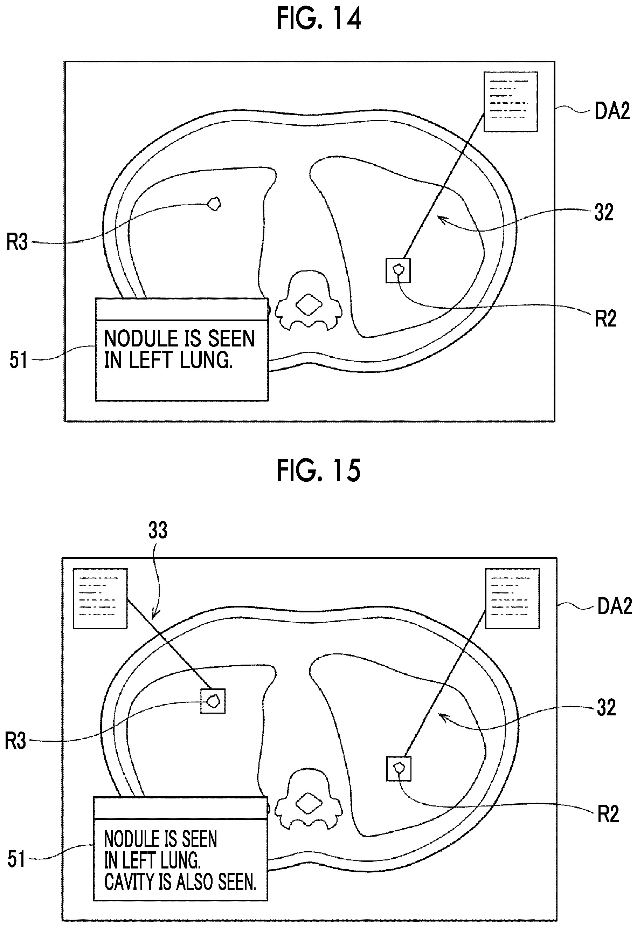

In this case, the display control unit 23 displays only the annotation 32 for the region of interest R2 and hides the annotation 33 for the region of interest R3. Accordingly, as shown in Fig. 14, the comment-on-findings input window 51 is displayed in the display area 43A, the annotation 33 added to the region of interest R3 is hidden based on the comment on findings of "a nodule is seen in the left lung" input in the comment-on-findings input window 51, and only the annotation 32 added to the region of interest R2 is displayed.

It is assumed that the radiologist further inputs a comment on findings of "a cavity is also seen" in the comment-on-findings input window 51. In this case, the specifying unit 24 analyzes the newly input comment on findings and specifies the term "cavity". In this case, as shown in Fig. 15, in a case where the text 33B of the annotation 33 added to the region of interest R3 includes a description regarding the cavity, the annotation 33 added to the region of interest R3 may be further displayed.

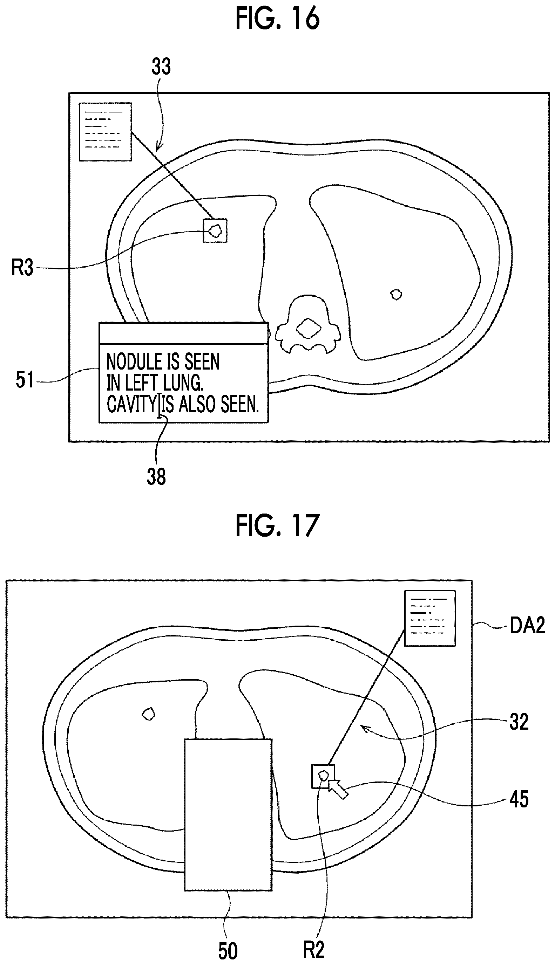

In this case, in a case where an input cursor 38 is located at the position of "a cavity is also seen" in the comment-on-findings input window 51, as shown in Fig. 16, the display control unit 23 may display the annotation 33 added to the region of interest R3 corresponding to the comment on findings of "a cavity is also seen", and hide the annotation 32 added to the region of interest R2. In addition, in a case where the input cursor 38 is located in the comment on findings of "a nodule is seen in the left lung", the display control unit 23 may display the annotation 32 added to the region of interest R2 corresponding to the comment on findings of "a nodule is seen in the left lung", and hide the annotation 33 added to the region of interest R3.

In a case where the operation of inputting the comment on findings is stopped, the display control unit 23 may continue to display the annotation or may hide the annotation. Alternatively, the annotation may be displayed for a predetermined time and then hidden.

Further, the radiologist may perform an operation of inputting a property in a case where an abnormal shadow is found in the target image G1. In this case, the radiologist performs an operation of displaying the property input window 50 shown in Fig. 7 in a pop-up manner by specifying a found abnormal shadow with the mouse cursor 45 and right-clicking the specified abnormal shadow. The specifying unit 24 specifies the region of interest where the mouse cursor 45 is located as the region of attention based on the detection result of the operation. For example, in a case where an operation of inputting properties to the region of interest R2 is performed in the target image G1 (tomographic image DA2) on which the annotations 32 and 33 are superimposed and displayed as shown in Fig. 10, the radiologist moves the mouse cursor 45 to the region of interest R2 and right-clicks. Upon detecting this operation, the specifying unit 24 specifies the region of interest R2 where the mouse cursor 45 is located as the region of attention.

In this case, as shown in Fig. 17, the display control unit 23 hides the annotation 33 added to the region of interest R3 in the target image G0 (the tomographic image DA2), and displays only the annotation 32 added to the region of interest R2. The radiologist can see the annotation 32 displayed for the region of interest R2 and add the missing property, or correct the erroneous property.

In addition, in a case where the operation of inputting the property is stopped, the display control unit 23 may continue to display the annotation or may hide the annotation. Alternatively, the annotation may be displayed for a predetermined time and then hidden.

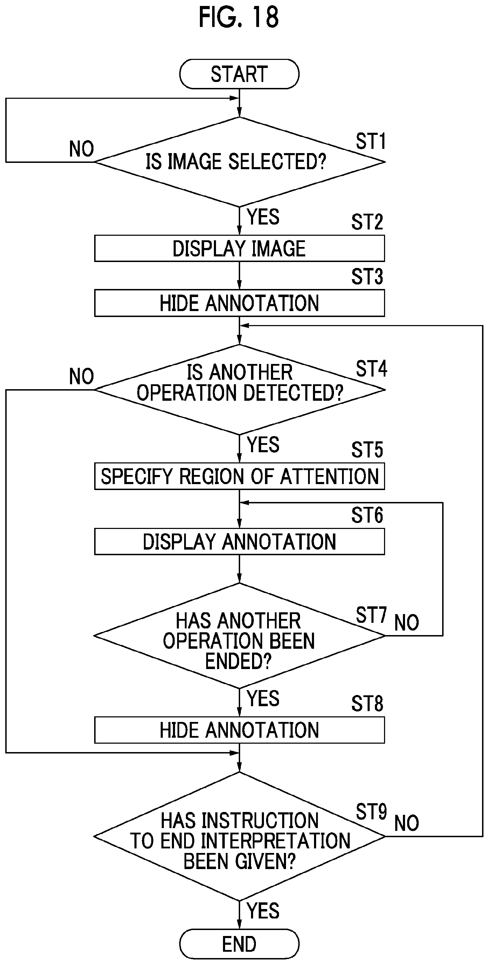

Next, a process performed in the present embodiment will be described. Fig. 18 is a flowchart showing a process performed in the present embodiment. It is assumed that a plurality of images including the target image G0 and the past image GP0 of the patient to be examined are acquired from the image server 5 and are saved in the storage 13. Further, it is assumed that the display control unit 23 displays the display screen 40 on the display 14, the examination list is selected on the display screen 40, and the image related to the selected examination is displayed in the thumbnail image area 42. In the thumbnail image area 42, in a case where an image to be displayed in the image display area 43 is selected (Step ST1; affirmative), the display control unit 23 displays the selected image in the image display area 43 (Step ST2). At this time, the display control unit 23 first hides the annotation even on the image in which the region of interest is detected (Step ST3).

In this state, the radiologist can perform operations such as performing a paging operation or the like, inputting a comment on findings, or adding a property tag to the newly found abnormal shadow.

Next, the specifying unit 24 determines whether or not another operation related to the display of the target image or another operation related to the interpretation of the image is detected (another operation detection; Step ST4). In a case where the determination in Step ST4 is affirmative, the specifying unit 24 specifies a region of attention among a plurality of regions of interest based on specification information including other information that is a detection result of another operation (Step ST5).

The display control unit 23 switches to display the annotation for the specified region of attention (Step ST6). Accordingly, the radiologist can interpret the region of interest with reference to the displayed annotation. In addition, it is possible to perform operations such as inputting a comment on findings or adding a property tag to the newly found abnormal shadow. The input comment on findings is saved in the storage 13 as an interpretation report. Further, the property tag is added to the target image G0 as tag information.

Subsequently, the display control unit 23 determines whether or not another operation has been ended (Step ST7), and in a case where the determination in Step ST7 is negative, the process returns to Step ST6. In a case where the determination in Step ST7 is affirmative, the annotation is switched to be hidden (Step ST8). In a case where the determinations in step ST4 are negative, and in a case where, after Step ST8, it is determined whether or not an instruction to end interpretation has been given (Step ST9) and the determination in Step ST9 is negative, the process returns to Step ST4. In a case where the determination in Step ST9 is affirmative, the process ends. The interpretation report generated by the interpretation of the target image G0 and the target image G0 to which the property tag is added are transferred to the report server 7. In the report server 7, the transferred interpretation report is saved together with the target image G0.

In a case where the determination in Step ST7 is affirmative, the annotation may be switched to be hidden after a predetermined time has elapsed. Alternatively, the display of the annotation may be continued. In a case where the display of the annotation is continued, another operation is detected, the region of attention is specified according to the detected operation, and the annotation related to the specified region of attention is displayed.

In this way, in the present embodiment, at least one region of attention is specified and the annotation for the specified region of attention is switched to be displayed based on specification information including other information other than the information dedicated to specifying the region of attention that the user pays attention to. Therefore, the radiologist can refer only to the annotation about the region of attention without performing a troublesome operation.

In particular, by hiding the annotations related to the region of interest other than the region of attention, it is possible to prevent the interpretation of the region of interest of attention from being hindered by the annotation about the region of interest that is not of attention.

In addition, by specifying the region of attention based on the detection results of other operations related to the display of the medical image or the interpretation of the medical image, the radiologist can specify the region of attention and display an annotation related to the region of attention without performing a dedicated operation for specifying the region of attention.

In the above embodiment, all the annotations are hidden in a case where the target image G0 is displayed, but the present disclosure is not limited thereto. In a case where the target image G0 is displayed, all the annotations may be displayed. In this case, in a case where the region of attention is specified based on specification information such as a detection result of another operation, it is sufficient that the display control unit 23 displays only the annotations related to the region of attention by switching to hide the annotations related to the region of interest other than the region of attention.

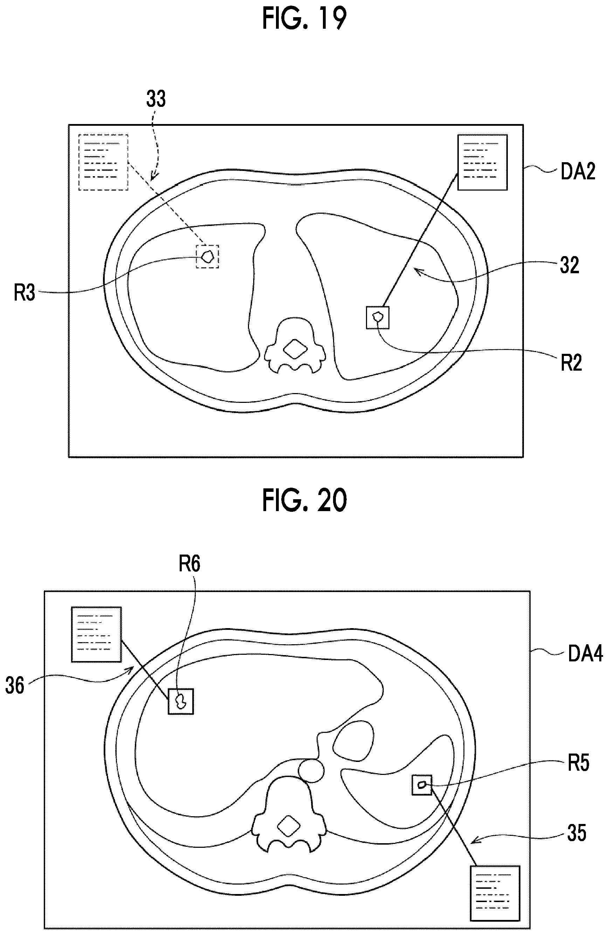

In addition, in the above-described embodiment, the annotation related to the region of attention is displayed and the annotation related to the region of interest other than the region of attention is hidden, but the present disclosure is not limited thereto. Annotations related to regions of interest other than the region of attention may be weakly displayed. Here, the "weak display" refers to making the degree of display weaker than that of the displayed annotation, such as increasing the transparency of the annotation, displaying the annotation with a broken line, making the density lower than that of the displayed annotation, making the color lighter, and the like. For example, in the tomographic image DA2 shown in Fig. 10, in a case where the region of interest R2 is specified as the region of attention, as shown in Fig. 19, the annotation 32 related to the region of interest R2 may be displayed and the annotation 33 related to the region of interest R3 may be displayed with a broken line.

In addition, in the above-described embodiment, the detection result of another operation is used as other information included in the specification information, but the present disclosure is not limited thereto. The region of attention may be specified by using the observation condition of the target image G0 as other information. Here, in a case where the target image G0 is a CT image, a window level (WL) and a window width (WW) are used as observation conditions in the case of displaying the CT image. WL is a CT value that is the center of the part to be observed in the gradation displayable by the display 14 in a case where the CT image is displayed on the display 14. WW is a width between a lower limit value and an upper limit value of the CT value of the part to be observed.

As such observation conditions, there are known observation conditions according to parts, such as a mediastinum condition that facilitates observation of bones and abdominal organs and a lung field condition that facilitates observation of lung fields. In addition to CT images, other medical images such as MRI images and radiation images acquired by simple radiography are also displayed under observation conditions that facilitate observation of specific parts. Therefore, in the specifying unit 24, an organ to be observed may be specified according to the observation condition, and the region of interest included in the specified organ may be specified as the region of attention.

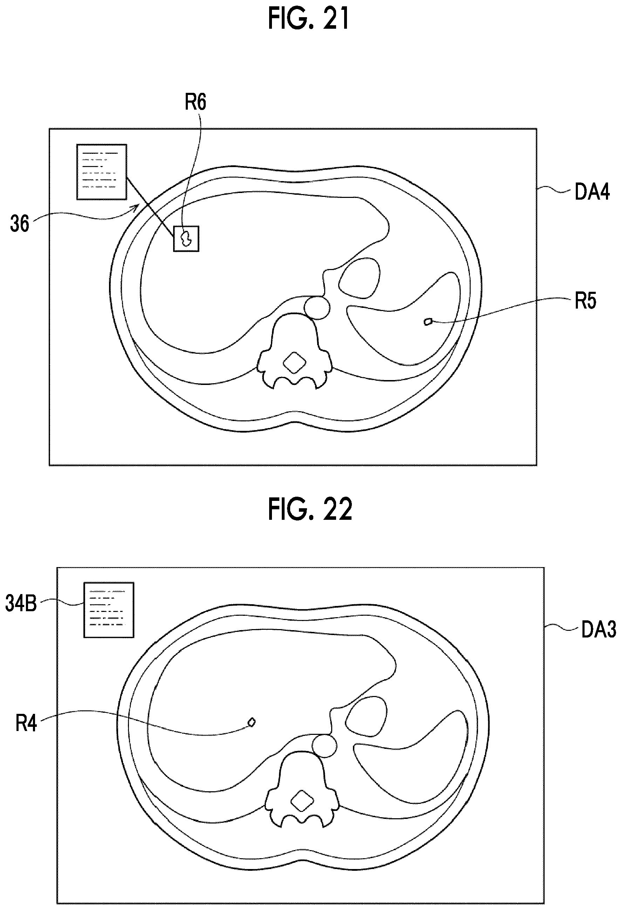

For example, in a tomographic image DA4 including a part of the liver and the left lung as shown in Fig. 20, it is assumed that a region of interest R5 is detected in the left lung and a region of interest R6 is detected in the liver. Further, it is assumed that an annotation 35 is added to the region of interest R5 of the left lung and an annotation 36 is added to the region of interest R6 of the liver. In this case, in a case where the observation condition is the mediastinum condition, the specifying unit 24 specifies the region of interest R6 of the liver as the region of attention. Therefore, as shown in Fig. 21, the display control unit 23 displays the annotation 36 added to the region of interest R6 and hides the annotation 35 added to the region of interest R5.

In addition, although the annotation related to the region of attention is displayed in the above-described embodiment, the annotation superimposed on the region of attention may be switched to be hidden in the annotation related to the region of attention. For example, as shown in Fig. 22, in a case where the region of interest R4 included in the tomographic image DA3 is specified as the region of attention, in the annotation 34, the figure 34A and the leader line 34C surrounding the region of interest R4 may be hidden, and only the text 34B outside the region of the human body may be displayed.

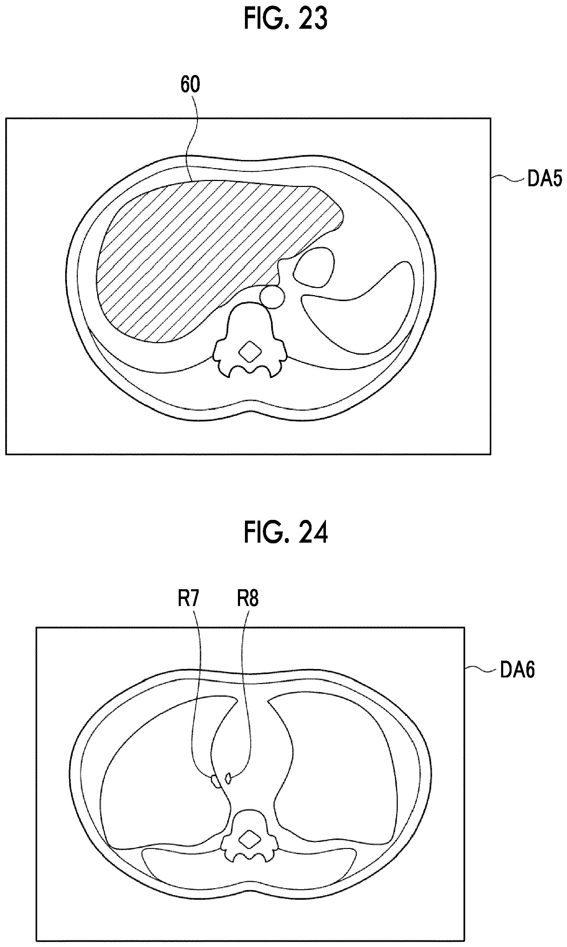

Further, in the above-described embodiment, the annotation is used as relevant information, but the present disclosure is not limited thereto. In addition to the annotation, examples of relevant information related to the region of interest include a region extraction result of a structure included in the target image G0 or the like. For example, as shown in Fig. 23, a mask 60 for a region of the liver included in the target image G0 may be superimposed and displayed on a tomographic image DA5 of the target image as a region extraction result. In this case, in a case where the region of interest is the liver and the relevant information is the mask 60 for the liver, the mask 60 may be displayed on the liver based on detection results of another operation related to the display of the image or another operation related to the interpretation of the image.

In addition, in the above-described embodiment, the display and hiding of the annotation only for the target image G0 (G1) displayed in the display area 43A has been described, but in a case where annotations are also added to the images displayed in the other display areas 43B to 43D, the annotations may be switched between display and hiding in the same manner as the display area 43A.

In addition, in the above-described embodiment, the region of attention is specified as the specification information based on information other than the information dedicated to specifying the region of attention that the user pays attention to, but the specification information is not limited thereto. The detection result of a dedicated operation for specifying the region of attention, such as a mouse click by the radiologist, may also be used as the specification information. For example, as shown in Fig. 24, it is assumed that the pleura of the lung field included in a tomographic image DA6 includes a region of interest R7, and the mediastinum includes a region of interest R8 adjacent to the region of interest R7. In this case, in a case where the radiologist selects the region of interest R7 in the lung field by clicking the mouse, the radiologist may erroneously select the region of interest R8 adjacent to the region of interest R7. In such a case, the specifying unit 24 may use the detection result of the operation of the radiologist clicking the mouse to select the region of interest and the observation condition of the target image G0 including the tomographic image DA6 as specification information, and display the annotation.

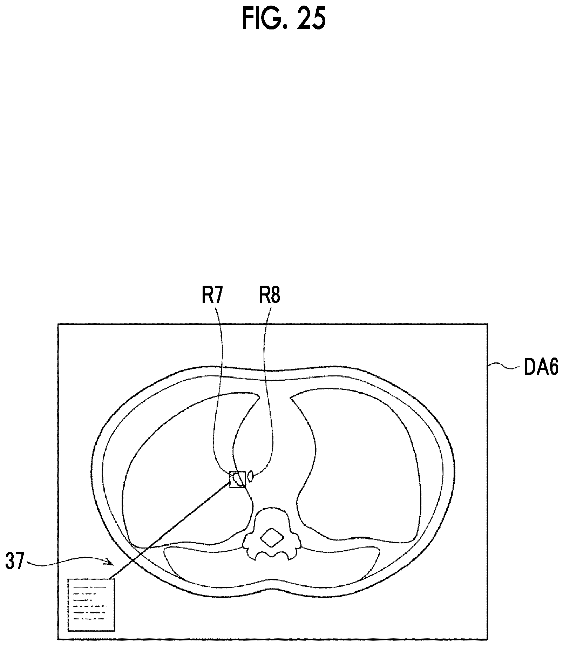

For example, in a case where the observation condition of the target image G0 is the lung field condition, the region of interest that the radiologist pays attention to is included in the lung field. Therefore, in a case where the observation condition is the lung field condition, the specifying unit 24 determines that the selected region of interest is the region of interest R7 included in the lung field, and specifies the region of interest R7 as the region of attention based on the detection results of the operation of selecting the observation condition and the region of attention. In this case, as shown in Fig. 25, an annotation 37 added to the region of interest R7 is displayed. Also, in a case where the observation condition is the mediastinum condition, the specifying unit 24 determines that the selected region of interest is the region of interest R8 included in the mediastinum, and specifies the region of interest R8 as the region of attention based on the detection results of the operation of selecting the observation condition and the region of attention. In this case, it is sufficient to display only the annotation added to the region of interest R8.

Further, in the above-described embodiment, a plurality of images are displayed on the display screen 40 at the same time, but the present disclosure is not limited thereto. The technology of the present disclosure can also be applied to a case where only one image is displayed on the display screen 40.

Further, in the above-described embodiment, the image display apparatus included in the interpretation WS 3 according to the present embodiment comprises the analysis unit 22 so that the interpretation WS 3 analyzes the target image, but the present disclosure is not limited thereto. The target image may be analyzed by an external analysis device different from the interpretation WS 3, and the analysis result may be acquired by the interpretation WS 3. In addition, an annotation may already be superimposed on the image saved in the image server 5. Even in such a case, as in the above-described embodiment, it is possible to display the image on which the annotation is superimposed in the interpretation WS 3 and to switch to hide the annotation based on detection results of another operation related to the display of the image or another operation related to the interpretation of the image.

In each of the above embodiments, for example, as hardware structures of processing units that execute various kinds of processing, such as the image acquisition unit 21, the analysis unit 22, the display control unit 23, and the specifying unit 24, various processors shown below can be used. As described above, the various processors include a programmable logic device

(PLD) as a processor of which the circuit configuration can be changed after manufacture, such as a field programmable gate array (FPGA), a dedicated electrical circuit as a processor having a dedicated circuit configuration for executing specific processing such as an application specific integrated circuit (ASIC), and the like, in addition to the CPU as a general-purpose processor that functions as various processing units by executing software (programs).

One processing unit may be configured by one of the various processors, or may be configured by a combination of the same or different kinds of two or more processors (for example, a combination of a plurality of FPGAs or a combination of the CPU and the FPGA). In addition, a plurality of processing units may be configured by one processor.

As an example in which a plurality of processing units are configured by one processor, first, there is a form in which one processor is configured by a combination of one or more CPUs and software as typified by a computer, such as a client or a server, and this processor functions as a plurality of processing units. Second, there is a form in which a processor for realizing the function of the entire system including a plurality of processing units via one integrated circuit (IC) chip as typified by a system on chip (SoC) or the like is used. As described above, various processing units are configured by using one or more of the above-described various processors as hardware structures.

Furthermore, as the hardware structure of the various processors, more specifically, an electrical circuit (circuitry) in which circuit elements such as semiconductor elements are combined can be used.

### Explanation of References

1: medical information system
2: imaging apparatus
3: interpretation WS
4: medical care WS
5: image server
6: image DB
7: report server
8: report DB
10: network
11: CPU
12: image display program
13: storage
14: display
15: input device
16: memory
17: network I/F
18: bus
20: image display apparatus
21: image acquisition unit
22: analysis unit
23: display control unit
24: specifying unit
31 to 37: annotation
32A, 33A, 34A: figure
32B, 33B, 34B: text
32C, 33C, 34C: leader line
40: display screen
41: examination list area
42: thumbnail image area
43: image display area
43A to 43D: display area
45: mouse cursor
50: property input window
51: comment-on-findings input window
60: mask
C1 to C6: thumbnail image
Dj, DA1 to DA6: tomographic image
G0, G1, G2: target image
GA1 to GA3: tomographic image group
GP1 to GP4: past image
R1 to R8: region of interest

## Claims

1. An image display apparatus (20) comprising at least one processor (11),
wherein the processor (11) is configured to:
superimpose and display (ST2) relevant information related to each of a plurality of regions of interest included in a medical image on the medical image to be switchable between display and hiding, wherein the medical image is a three-dimensional image including a plurality of tomographic images;
specify (ST5) at least one region of attention based on specification information including other information other than information dedicated to specifying regions of attention that a user pays attention to among the plurality of regions of interest, wherein the other information is a detection result of another operation related to display or interpretation of the medical image; and
switch (ST6) to display relevant information related to the specified region of attention;
wherein the other operation is a paging operation on the tomographic image, and
the processor (11) is configured to specify a region of interest included in the tomographic image being paging-operated as the region of attention.

2. The image display apparatus (20) according to claim 1,
wherein the processor (11) is configured to switch to hide or weakly display the relevant information related to the regions of interest other than the region of attention.

3. The image display apparatus (20) according to claim 1 or 2, wherein the other information is additionally an observation condition of the medical image.

4. The image display apparatus (20) according to claim 1,
wherein the processor (11) is configured to:
display (ST2) the medical image in which all relevant information is hidden; and
display the relevant information related to the region of attention in a case where the region of attention is specified based on the detection result of the other operation.

5. The image display apparatus (20) according to any one of claims 1 to 4,
wherein the other operation is additionally an operation of inputting a comment on findings related to the medical image, and
the processor (11) is configured to specify a region of interest related to a term included in the comment on findings as the region of attention.

6. The image display apparatus (20) according to any one of claims 1 to 5,
wherein the other operation is additionally an operation of further adding the relevant information to the region of interest included in the medical image, and
the processor (11) is configured to specify the region of interest to which the relevant information is added as the region of attention.

7. The image display apparatus (20) according to any one of claims 1 to 6,
wherein the other operation is additionally an operation of changing a size of the medical image with reference to a designated region of interest, and
the processor (11) is configured to specify the designated region of interest as the region of attention.

8. The image display apparatus (20) according to any one of claims 1 to 7,
wherein the processor (11) is configured to switch to hide the relevant information superimposed on the region of attention by the other operation.

9. The image display apparatus (20) according to any one of claims 1 to 8,
wherein the specification information includes only the other information.

10. The image display apparatus (20) according to any one of claims 1 to 9,
wherein the relevant information includes an annotation related to the region of interest.

11. The image display apparatus (20) according to claim 10,
wherein the annotation includes at least one of a recognition result of the region of interest or character information related to the region of interest.

12. The image display apparatus (20) according to claim 10 or 11,
wherein the region of interest is a lesion or an organ included in the medical image.

13. An image display method comprising:
superimposing and displaying (ST2) relevant information related to each of a plurality of regions of interest included in a medical image on the medical image to be switchable between display and hiding, wherein the medical image is a three-dimensional image including a plurality of tomographic images;
specifying (ST5) at least one region of attention based on specification information including other information other than information dedicated to specifying regions of attention that a user pays attention to among the plurality of regions of interest, wherein the other information is a detection result of another operation related to display or interpretation of the medical image; and
switching (ST6) to display relevant information related to the specified region of attention;
wherein the other operation is a paging operation on the tomographic image, and
the specified a region of interest is included in the tomographic image being paging-operated as the region of attention.

14. A computer-readable storage medium that stores an image display program causing a computer to execute:
a procedure of superimposing and displaying (ST2) relevant information related to each of a plurality of regions of interest included in a medical image on the medical image to be switchable between display and hiding, wherein the medical image is a three-dimensional image including a plurality of tomographic images;
a procedure of specifying (ST5) at least one region of attention based on specification information including other information other than information dedicated to specifying regions of attention that a user pays attention to among the plurality of regions of interest, wherein the other information is a detection result of another operation related to display or interpretation of the medical image; and
a procedure of switching to display (ST6) relevant information related to the specified region of attention;
wherein the other operation is a paging operation on the tomographic image, and
the specified a region of interest is included in the tomographic image being paging-operated as the region of attention.

## Patentansprüche

1. Bildanzeigevorrichtung (20), die mindestens einen Prozessor (11) umfasst,
wobei der Prozessor (11) so konfiguriert ist, dass er:
relevante Informationen, die sich auf jeden von mehreren Bereichen von Interesse, die in einem medizinischen Bild enthalten sind, beziehen, dem medizinischen Bild, das zwischen Anzeigen und Ausblenden umzuschalten ist, überlagert und anzeigt (ST2), wobei das medizinische Bild ein dreidimensionales Bild, das mehrere Tomographiebilder enthält, ist;
mindestens einen Aufmerksamkeitsbereich auf der Grundlage von Spezifikationsinformationen, die andere Informationen als Informationen, die zum Spezifizieren von Aufmerksamkeitsbereichen, denen ein Benutzer Aufmerksamkeit schenkt, dediziert sind, enthalten, unter den mehreren Bereichen von Interesse spezifiziert (ST5), wobei die anderen Informationen ein Detektionsergebnis eines anderen Vorgangs in Bezug auf Anzeige oder Auswertung des medizinischen Bildes sind; und
umschaltet (ST6), um relevante Informationen, die sich auf den spezifizierten Aufmerksamkeitsbereich beziehen, anzuzeigen;
wobei der andere Vorgang ein Paging-Vorgang an dem Tomographiebild ist und der Prozessor (11) so konfiguriert ist, dass er einen Bereich von Interesse, der in dem Tomographiebild, an dem Paging durchgeführt wird, enthalten ist, als den Aufmerksamkeitsbereich spezifiziert.

2. Bildanzeigevorrichtung (20) nach Anspruch 1,
wobei der Prozessor (11) so konfiguriert ist, dass er umschaltet, um die relevanten Informationen, die sich auf die anderen Bereiche von Interesse als der Aufmerksamkeitsbereich beziehen, zu verbergen oder schwach anzuzeigen.

3. Bildanzeigevorrichtung (20) nach Anspruch 1 oder 2,
wobei die anderen Informationen darüber hinaus
eine Beobachtungsbedingung des medizinischen Bildes sind.

4. Bildanzeigevorrichtung (20) nach Anspruch 1,
wobei der Prozessor (11) so konfiguriert ist, dass er:
das medizinische Bild, in dem alle relevanten Informationen verborgen sind, anzeigt (ST2); und
die relevanten Informationen, die sich auf den Aufmerksamkeitsbereich beziehen, in einem Fall, in dem der Aufmerksamkeitsbereich auf der Grundlage des Detektionsergebnisses des anderen Vorgangs spezifiziert wird, anzeigt.

5. Bildanzeigevorrichtung (20) nach einem der Ansprüche 1 bis 4,
wobei der andere Vorgang darüber hinaus
ein Vorgang des Eingebens eines Kommentars zu Befunden, die sich
auf das medizinische Bild beziehen, ist, und
der Prozessor (11) so konfiguriert ist, dass er einen Bereich von Interesse, der sich auf einen Begriff, der in dem Kommentar zu Befunden enthalten ist, bezieht, als den Aufmerksamkeitsbereich spezifiziert.

6. Bildanzeigevorrichtung (20) nach einem der Ansprüche 1 bis 5,
wobei der andere Vorgang darüber hinaus
ein Vorgang des weiteren Hinzufügens der relevanten Informationen zu dem Bereich von Interesse, der in dem medizinischen Bild enthalten ist, ist, und der Prozessor (11) so konfiguriert ist, dass er den Bereich von Interesse, zu dem die relevanten Informationen hinzugefügt werden, als den Aufmerksamkeitsbereich spezifiziert.

7. Bildanzeigevorrichtung (20) nach einem der Ansprüche 1 bis 6,
wobei der andere Vorgang darüber hinaus
ein Vorgang des Änderns einer Größe des medizinischen Bildes
unter Bezugnahme auf einen designierten Bereich von Interesse ist, und
der Prozessor (11) so konfiguriert ist, dass er den designierten Bereich von Interesse als den Aufmerksamkeitsbereich spezifiziert.

8. Bildanzeigevorrichtung (20) nach einem der Ansprüche 1 bis 7,
wobei der Prozessor (11) so konfiguriert ist, dass er umschaltet, um die relevanten Informationen, die dem Aufmerksamkeitsbereich überlagert sind, durch den anderen Vorgang zu verbergen.

9. Bildanzeigevorrichtung (20) nach einem der Ansprüche 1 bis 8,
wobei die Spezifikationsinformationen nur die anderen Informationen enthalten.

10. Bildanzeigevorrichtung (20) nach einem der Ansprüche 1 bis 9,
wobei die relevanten Informationen eine Beschriftung, die sich auf den Bereich von Interesse bezieht, enthalten.

11. Bildanzeigevorrichtung (20) nach Anspruch 10,
wobei die Beschriftung mindestens eines von einem Erkennungsergebnis des Bereichs von Interesse und Zeicheninformationen, die sich auf den Bereich von Interesse beziehen, enthält.

12. Bildanzeigevorrichtung (20) nach Anspruch 10 oder 11,
wobei der Bereich von Interesse eine Läsion oder ein Organ ist, das in dem medizinischen Bild enthalten ist.

13. Bildanzeigeverfahren, umfassend:
Überlagern von relevanten Informationen, die sich auf jeden von mehreren Bereichen von Interesse, die in einem medizinischen Bild enthalten sind, beziehen, mit dem medizinischen Bild, das zwischen Anzeigen und Ausblenden umzuschalten ist, und Anzeigen davon (ST2), wobei das medizinische Bild ein dreidimensionales Bild, das mehrere Tomographiebilder enthält, ist;
Spezifizieren (ST5) mindestens eines Aufmerksamkeitsbereichs auf der Grundlage von Spezifikationsinformationen, die andere Informationen als Informationen, die zum Spezifizieren von Aufmerksamkeitsbereichen, denen ein Benutzer Aufmerksamkeit schenkt, dediziert sind, enthalten, unter den mehreren Bereichen von Interesse, wobei die anderen Informationen ein Detektionsergebnis eines anderen Vorgangs in Bezug auf Anzeige oder Auswertung des medizinischen Bildes sind; und
Umschalten (ST6), um relevante Informationen, die sich auf den spezifizierten Aufmerksamkeitsbereich beziehen, anzuzeigen;
wobei der andere Vorgang ein Paging-Vorgang an dem Tomographiebild ist und der spezifizierte Bereich von Interesse in dem Tomographiebild, an dem Paging durchgeführt wird, als der Aufmerksamkeitsbereich enthalten ist.

14. Computerlesbares Speichermedium, das ein Bildanzeigeprogramm speichert, das einen Computer veranlasst, auszuführen:
ein Verfahren des Überlagerns von relevanten Informationen, die sich auf jeden von mehreren Bereichen von Interesse, die in einem medizinischen Bild enthalten sind, beziehen, mit dem medizinischen Bild, das zwischen Anzeigen und Ausblenden umzuschalten ist, und des Anzeigens davon (ST2), wobei das medizinische Bild ein dreidimensionales Bild, das mehrere Tomographiebilder enthält, ist;
ein Verfahren des Spezifizierens (ST5) mindestens eines Aufmerksamkeitsbereichs auf der Grundlage von Spezifikationsinformationen, die andere Informationen als Informationen, die zum Spezifizieren von Aufmerksamkeitsbereichen, denen ein Benutzer Aufmerksamkeit schenkt, dediziert sind, enthalten, unter den mehreren Bereichen von Interesse, wobei die anderen Informationen ein Detektionsergebnis eines anderen Vorgangs in Bezug auf Anzeige oder Auswertung des medizinischen Bildes sind; und
ein Verfahren des Umschaltens (ST6), um relevante Informationen, die sich auf den spezifizierten Aufmerksamkeitsbereich beziehen, anzuzeigen;
wobei der andere Vorgang ein Paging-Vorgang an dem Tomographiebild ist, und
der spezifizierte Bereich von Interesse in dem Tomographiebild, an dem Paging durchgeführt wird, als der Aufmerksamkeitsbereich enthalten ist.

## Revendications

1. Appareil d'affichage d'images (20) comprenant au moins un processeur (11),
dans lequel le processeur (11) est configuré pour :
superposer et afficher (ST2) des informations pertinentes liées à chacune d'une pluralité de régions d'intérêt incluses dans une image médicale sur l'image médicale, de manière à pouvoir commuter entre l'affichage et la dissimulation, dans lequel l'image médicale est une image tridimensionnelle comprenant une pluralité d'images tomographiques ;
spécifier (ST5) au moins une région d'attention sur la base d'informations de spécification comprenant d'autres informations que celles dédiées à la spécification de régions d'attention auxquelles un utilisateur prête attention, parmi la pluralité de régions d'intérêt, dans lequel les autres informations sont un résultat de détection d'une autre opération liée à l'affichage ou à l'interprétation de l'image médicale ; et
commuter (ST6) pour afficher les informations pertinentes liées à la région d'attention spécifiée ;
dans lequel l'autre opération est une opération de pagination sur l'image tomographique et le processeur (11) est configuré pour spécifier une région d'intérêt incluse dans l'image tomographique en cours de pagination comme la région d'attention.

2. Appareil d'affichage d'images (20) selon la revendication 1,
dans lequel le processeur (11) est configuré pour commuter afin de dissimuler ou afficher faiblement les informations pertinentes liées aux régions d'intérêt autres que la région d'attention.

3. Appareil d'affichage d'images (20) selon la revendication 1 ou la revendication 2,
dans lequel les autres informations sont en outre
une condition d'observation de l'image médicale.

4. Appareil d'affichage d'images (20) selon la revendication 1,
dans lequel le processeur (11) est configuré pour :
afficher (ST2) l'image médicale dans laquelle toutes les informations pertinentes sont dissimulées ; et
afficher les informations pertinentes liées à la région d'attention dans un cas où la région d'attention est spécifiée sur la base du résultat de détection de l'autre opération.

5. Appareil d'affichage d'images (20) selon l'une quelconque des revendications 1 à 4,
dans lequel l'autre opération est en outre
une opération de saisie d'un commentaire sur des résultats liés
à l'image médicale, et
le processeur (11) est configuré pour spécifier une région d'intérêt liée à un terme inclus dans le commentaire sur des résultats en tant que les région d'attention.

6. Appareil d'affichage d'images (20) selon l'une quelconque des revendications 1 à 5,
dans lequel l'autre opération est en outre
une opération d'ajout supplémentaire des informations pertinentes
à la région d'intérêt incluse dans l'image médicale, et
le processeur (11) est configuré pour spécifier la région d'intérêt à laquelle les informations pertinentes sont ajoutées en tant que les région d'attention.

7. Appareil d'affichage d'images (20) selon l'une quelconque des revendications 1 à 6,
dans lequel l'autre opération est en outre
une opération de changement d'une taille de l'image médicale
en référence à une région d'intérêt désignée, et
le processeur (11) est configuré pour spécifier la région d'intérêt désignée comme la région d'attention.

8. Appareil d'affichage d'images (20) selon l'une quelconque des revendications 1 à 7,
dans lequel le processeur (11) est configuré pour commuter afin de dissimuler les informations pertinentes superposées sur la région d'attention par l'autre opération.

9. Appareil d'affichage d'images (20) selon l'une quelconque des revendications 1 à 8,
dans lequel les informations de spécification comprennent uniquement les autres informations.

10. Appareil d'affichage d'images (20) selon l'une quelconque des revendications 1 à 9,
dans lequel les informations pertinentes comprennent une annotation liée à la région d'intérêt.

11. Appareil d'affichage d'images (20) selon la revendication 10,
dans lequel l'annotation comprend au moins l'un d'un résultat de reconnaissance de la région d'intérêt et d'informations de caractère liées à la région d'intérêt.

12. Appareil d'affichage d'images (20) selon la revendication 10 ou la revendication 11,
dans lequel la région d'intérêt est une lésion ou un organe inclus dans l'image médicale.

13. Procédé d'affichage d'images comprenant :
superposer et afficher (ST2) des informations pertinentes liées à chacune d'une pluralité de régions d'intérêt incluses dans une image médicale sur l'image médicale, de manière à pouvoir commuter entre l'affichage et la dissimulation, dans lequel l'image médicale est une image tridimensionnelle comprenant une pluralité d'images tomographiques ;
spécifier (ST5) au moins une région d'attention sur la base d'informations de spécification comprenant d'autres informations que celles dédiées à la spécification de régions d'attention auxquelles un utilisateur prête attention, parmi la pluralité de régions d'intérêt, dans lequel les autres informations sont un résultat de détection d'une autre opération liée à l'affichage ou à l'interprétation de l'image médicale ; et commuter (ST6) pour afficher les informations pertinentes liées à la région d'attention spécifiée ;
dans lequel l'autre opération est une opération de pagination sur l'image tomographique et la région d'intérêt spécifiée est incluse dans l'image tomographique en cours de pagination comme la région d'attention.

14. Support de stockage lisible par ordinateur qui stocke un programme d'affichage d'images amenant un ordinateur à exécuter :
une procédure de superposition et d'affichage (ST2) d'informations pertinentes liées à chacune d'une pluralité de régions d'intérêt incluses dans une image médicale sur l'image médicale, de manière à pouvoir commuter entre l'affichage et la dissimulation, dans lequel l'image médicale est une image tridimensionnelle comprenant une pluralité d'images tomographiques ;
une procédure de spécification (ST5) au moins d'une région d'attention sur la base d'informations de spécification comprenant d'autres informations que celles dédiées à la spécification de régions d'attention auxquelles un utilisateur prête attention, parmi la pluralité de régions d'intérêt, dans lequel les autres informations sont un résultat de détection d'une autre opération liée à l'affichage ou à l'interprétation de l'image médicale ; et
une procédure de commutation pour afficher (ST6) des informations pertinentes liées à la région d'attention spécifiée ;
dans laquelle l'autre opération est une opération de pagination sur l'image tomographique, et
la région d'intérêt spécifiée est incluse dans l'image tomographique en cours de pagination comme la région d'attention.
